# EUROPEAN PATENT APPLICATION

(11) **EP 4 624 655 A2**
(43) Date of publication of application: **01.10.2025**
(21) Application number: 25165768.0
(22) Date of filing: 24.03.2025
(51) Int. Cl.: D06N 3/00, B32B 23/10

(54) **HYPHA COMPOSITE MATERIAL**

(30) Priority: 26.03.2024 JP 2024049581
(71) Applicant: Seiko Epson Corporation, Tokyo 160-8801 (JP)
(72) Inventor: SAWAI, Takenori, Suwa-shi, 392-8502 (JP)
(74) Representative: Lewis Silkin LLP

(57) **Abstract**

Provided is a hypha composite material including: a fiber oriented in a first direction; and a mycelium, in which the first direction is a longitudinal direction of the fiber, and the fiber and the mycelium are integrated with cultured and grown hyphae. An aspect of the hypha composite material is a laminate in which hypha composite sheets are laminated. It is preferable that the mycelium is a mycelium of Lentinula edodes. In the laminate, at least a first hypha composite sheet and a second hypha composite sheet are laminated, and an orientation direction of a fiber of the first hypha composite sheet intersects an orientation direction of a fiber of the second hypha composite sheet.

## Description

The present application is based on, and claims priority from JP Application Serial Number 2024-049581, filed March 26, 2024, the disclosure of which is hereby incorporated by reference herein in its entirety.

### BACKGROUND

### 1. Technical Field

The present disclosure relates to a hypha composite material.

### 2. Related Art

Leather materials are used for various applications such as clothing and accessories. Examples of the leather material include tanned animal-derived leather from mammals such as cows and sheep, and reptiles such as snakes and crocodiles, and synthetic leather artificially manufactured to imitate the texture of leather.

In recent years, from the viewpoint of animal protection and the viewpoint of reducing environmental load, there has been a demand for alternative materials to replace animal-derived materials and materials which use a large amount of petroleum-derived raw materials, such as synthetic leather. As such alternative materials, materials that reproduce the appearance and texture of leather using plant-derived materials such as apple leather and mushroom leather are being considered, and such materials are known as vegan leather.

Mushroom leather, a type of vegan leather, is a material having a fine fibrous structure made from a mushroom mycelium. For example, JP-T-2022-534025 discloses a composite material containing a cultivated mycelium material and a bonding agent.

Since the alternative materials to the animal-derived leather and the synthetic leather are widely used in clothing, accessories, and even furniture, the alternative materials are required to have a unique texture and improved strength.

The present disclosure has been made in view of the above circumstances, and an object thereof is to provide a hypha composite material that can be used as vegan leather having a unique texture and improved strength.

### SUMMARY

That is, the present disclosure includes the following aspects.
[1] A hypha composite material includes: a fiber oriented in a first direction; and a mycelium, in which the first direction is a longitudinal direction of the fiber, and the fiber and the mycelium are integrated with cultured and grown hyphae.
[2] The hypha composite material according to [1], in which the hypha composite material is a laminate in which hypha composite sheets are laminated, at least a first hypha composite sheet and a second hypha composite sheet are laminated, and an orientation direction of a fiber of the first hypha composite sheet intersects an orientation direction of a fiber of the second hypha composite sheet.
[3] The hypha composite material according to [1] or [2], in which the fiber has a number average fiber length of 0.001 mm or more and 5.0 mm or less and a number average fiber diameter of 1.0 µm or more and 100.0 µm or less during defibration.
[4] The hypha composite material according to any one of [1] to [3], in which a ratio (A/B) of A to B is 10/90 to 90/10, where A is a content ratio of the fiber and B is a content ratio of the mycelium.
[5] The hypha composite material according to any one of [1] to [4], in which the hyphae have a number average fiber diameter of 1 µm or more and 10 µm or less, and the fiber has a number average fiber diameter of 11 µm or more and 30 µm or less.
[6] The hypha composite material according to any one of [1] to [5], in which the mycelium is a mycelium of Lentinula edodes.
[7] The hypha composite material according to any one of [1] to [6], further including: a plasticizer.
[8] The hypha composite material according to any one of [1] to [7], further including: a starch composite particle.
[9] The hypha composite material according to any one of [1] to [8], further including: a crosslinking agent.
[10] The hypha composite material according to any one of [1] to [9], in which a total content ratio of a plasticizer, a starch composite particle, and a crosslinking agent with respect to a total amount of the hypha composite material is 2.5% by mass or more and 80% by mass or less.
[11] The hypha composite material according to any one of [1] to [10], further including: a sugar alcohol as a plasticizer.
[12] The hypha composite material according to any one of [1] to [11], further including: a sugar alcohol as a plasticizer, in which the sugar alcohol is one or more selected from the group including sorbitol, erythritol, and D-mannitol.
[13] The hypha composite material according to any one of [1] to [12], further including: a plasticizer; and a starch composite particle, in which a content ratio of the plasticizer is 10% by mass or more and 80% by mass or less with respect to a total content (100% by mass) of the plasticizer and the starch composite particle.
[14] The hypha composite material according to any one of [1] to [13], further including a dicarboxylic acid as a crosslinking agent.
[15] The hypha composite material according to any one of [1] to [14], further including: a dicarboxylic acid as a crosslinking agent, in which the dicarboxylic acid is one or more selected from the group including succinic acid, adipic acid, and sebacic acid.
[16] The hypha composite material according to any one of [1] to [15], further including: a starch composite particle; and a crosslinking agent, in which a content ratio of the crosslinking agent is 1% by mass or more and 50% by mass or less with respect to a total content (100% by mass) of the starch composite particle and the crosslinking agent.
[17] The hypha composite material according to any one of [1] to [16], further including: a starch composite particle having an average particle diameter of 1 µm or more and 50 µm or less.
[18] The hypha composite material according to any one of [1] to [17], in which the fiber contains cellulose.

According to the present disclosure, it is possible to provide a hypha composite material that can be used as vegan leather having a unique texture and improved strength.

### BRIEF DESCRIPTION OF THE DRAWINGS

FIG. 1 is a schematic view illustrating an example of a laminate according to an embodiment.
FIG. 2 is a schematic view illustrating an example of an orientation device used for producing a hypha composite material according to the embodiment.

### DESCRIPTION OF EMBODIMENTS

### HYPHA COMPOSITE MATERIAL

One aspect of the present disclosure is a hypha composite material including a fiber oriented in a first direction and a mycelium. In the hypha composite material, the fiber and the mycelium are integrated with a hypha cultured and grown.

More specifically, in the hypha composite material according to the embodiment, the hypha is grown and cultured using the fiber as a scaffold, and the fiber and the mycelium are integrated. Since the fiber serving as the scaffold is oriented in the first direction, strength in a direction parallel to a fiber orientation is improved. Since the hypha is cultured and grown to fill gaps between the fibers and the fiber and the mycelium are integrated, strength in a direction intersecting with the fiber orientation is also improved. Here, the term "integrated" includes forms such as a state in which the fiber and the hypha are intertwined, a state in which the hypha is wound around the fiber, and a state in which the hypha is bonded to a surface of the fiber.

In the present specification, the term "hypha" is an elongated cell that constitutes a fungus. The hypha is elongated or branched by tip growth. The term "mycelium" is an aggregate of hyphae.

In the present specification, the term "cultured and grown" means that hypha is intentionally grown.

The hypha composite material according to the embodiment is formed of the mycelium in which the hypha is cultured and grown and grown by spontaneous growth. The hypha composite material formed of the mycelium grown by spontaneous growth can reproduce texture such as appearance and feel of suede which is leather made from tanned leather with short fluff. Various textures can be reproduced depending on processing methods.

In one aspect of the present disclosure, the hypha composite material is a single-layer hypha composite sheet.

In one aspect of the present disclosure, the hypha composite material is a laminate in which single-layer hypha composite sheets are laminated.

When the hypha composite material is a laminate, at least a first hypha composite sheet and a second hypha composite sheet are laminated, and an orientation direction of a fiber of the first hypha composite sheet intersects an orientation direction of a fiber of the second hypha composite sheet. The first hypha composite sheet and the second hypha composite sheet are each a single-layer hypha composite sheet as one aspect of the present disclosure.

In one aspect of the present disclosure, when the hypha composite material is a laminate, if the hypha composite sheet is used as a layer on the outermost surface, a sheet of another material may be used in an intermediate layer or a lower layer of the laminate. As a sheet of another material, vegan leather such as apple leather may be used, and a petroleum-based sheet may be used.

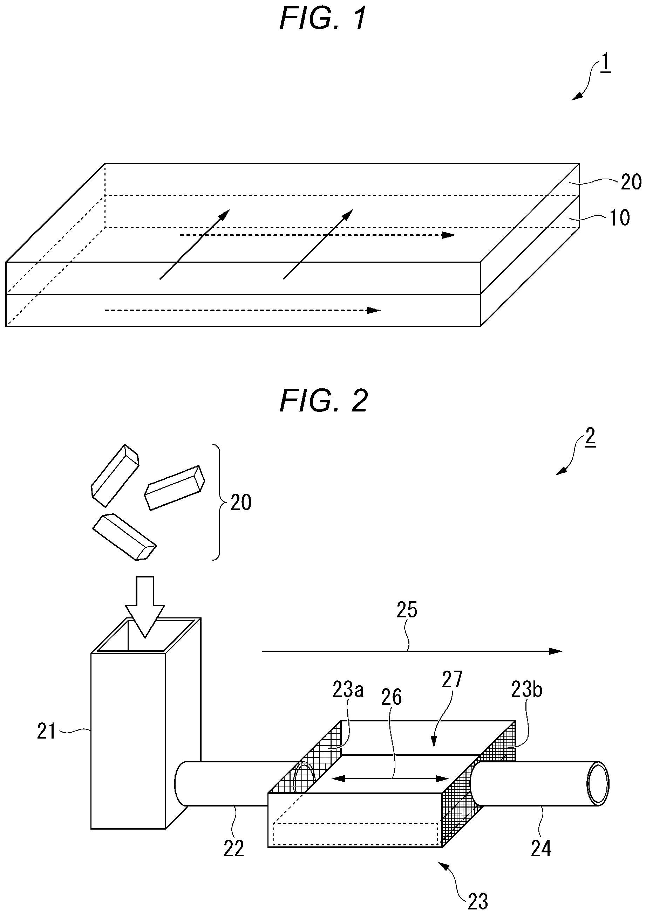

FIG. 1 illustrates a laminate 1 according to an aspect of the present disclosure. The laminate 1 is a laminate in which a first hypha composite sheet 10 and a second hypha composite sheet 20 are laminated. In FIG. 1, arrows indicated by broken lines indicate an orientation direction of a fiber of the first hypha composite sheet, and arrows indicated by solid lines indicate an orientation direction of a fiber of the second hypha composite sheet.

In the laminate 1, the orientation direction of the fiber of the first hypha composite sheet indicated by the broken lines intersects the orientation direction of the fiber of the second hypha composite sheet indicated by the solid lines.

Although the single-layer hypha composite sheet has strength, the single-layer hypha composite sheet tends to tear easily in a direction along the fiber orientation. When the first hypha composite sheet and the second hypha composite sheet are laminated in a form in which the orientation directions of the fibers intersect, the laminated hypha composite sheets complement each other in strength, thereby obtaining a hypha composite material having further improved strength.

When the hypha composite material is a laminate, it is sufficient that the single-layer hypha composite sheets are alternately laminated in the mode in which the orientation directions of the fibers intersect, and the number of laminations is not particularly limited and can be adjusted as appropriate depending on desired strength and texture.

### Fiber

The fiber forming the hypha composite material is oriented in the first direction. In the present specification, the first direction is a longitudinal direction of the fiber, and the longitudinal direction of the fiber means a direction along a straight line connecting both ends of the fiber.

Examples of the fiber include cellulose fibers, cotton fibers, rayon fibers, lyocell fibers, TENCEL (trademark) fibers, polypropylene fibers, and combinations thereof. Among them, fibers containing cellulose are preferably used from a viewpoint of using a plant-derived material.

A number average fiber length of the fiber at a time of defibration is preferably 0.001 mm or more and 5.0 mm or less, more preferably 0.002 mm or more and 3.0 mm or less, and still more preferably 0.003 mm or more and 2.0 mm or less.

When the number average fiber length of the fiber at the time of defibration is within the above ranges, the fiber is easily oriented in the first direction, a hypha composite material in which fibers are moderately intertwined is easily obtained, and mechanical strength of the hypha composite material can be particularly increased.

A number average fiber diameter of the fiber is preferably 1.0 µm or more and 100.0 µm or less, and more preferably 3.0 µm or more and 50.0 µm or less. When the number average fiber diameter of the fiber is within the above ranges, mechanical strength of the hypha composite material can be particularly increased.

In one aspect of the present disclosure, from a viewpoint of increasing the mechanical strength of the hypha composite material, a fiber diameter of the fiber is preferably larger than a fiber diameter of the hypha, a number average fiber diameter of the hyphae is preferably 1 µm or more and 10 µm or less, and the number average fiber diameter of the fiber is preferably 11 µm or more and 30 µm or less.

In one aspect of the present disclosure, a ratio (A/B) of A to B is 10/90 to 90/10, where A is a content ratio of the fiber, and B is a content ratio of the mycelium with respect to with respect to a total amount of the hypha composite material.

The ratio of A to B can be adjusted as appropriate depending on a desired texture.

For example, when the ratio (A/B) is in a range of 10/90 to 30/70 and the ratio of the mycelium is large, a fluffy, soft texture or a suede-like texture can be reproduced.

When the ratio (A/B) is in a range of 70/30 to 90/10 and the ratio of the mycelium is small, the texture of cowhide, sheepskin, snakeskin, crocodile leather, and the like can be reproduced.

### Mycelium

Examples of the mycelium include one or two or more of mycelia selected from the group including Lentinula edodes, Agaricus arvensis, Agrocybe brasiliensis, Amylomyces rouxii, Amylomyces species, Armillaria mellea, Aspergillus nidulans, Aspergillus niger, Aspergillus oryzae, Ceriporia lacerata, Coprinus comatus, Fibroporia vaillantii, Fistulina hepatica, Flammulina velutipes, Fomitopsis officinalis, Ganoderma sessile, Ganoderma tsugae, Hericium erinaceus, Hypholoma capnoides, Hypholoma sublaterium, Inonotus obliquus, Lactarius chrysorrheus, Macrolepiota procera, Morchella angusticeps, Myceliophthora thermophila, Neurospora crassa, Penicillium camembertii, Penicillium chrysogenum, Penicillium rubens, Phycomyces blakesleeanus, Pleurotus djamor, Pleurotus ostreatus, Polyporus squamosus, Psathyrella aquatica, Rhizopus microspores, Rhizopus oryzae, Schizophyllum commune, Streptomyces venezuelae, Stropharia rugosoannulata, Thielavia terrestris, and Ustilago maydis.

Among the above, from a viewpoint of improving the texture of the hypha composite material, the mycelium is preferably a mycelium of fungus that forms a fruiting body that is a sporophore. Examples of such mycelium include one or two or more types of mycelia selected from the group including Lentinula edodes, Agaricus arvensis, Agrocybe brasiliensis, Amylomyces rouxii, Amylomyces species, Armillaria mellea, Aspergillus nidulans, Ceriporia lacerata, Coprinus comatus, Fibroporia vaillantii, Fistulina hepatica, Flammulina velutipes, Fomitopsis officinalis, Ganoderma sessile, Ganoderma tsugae, Hericium erinaceus, Hypholoma capnoides, Hypholoma sublaterium, Inonotus obliquus, Lactarius chrysorrheus, Macrolepiota procera, Morchella angusticeps, Myceliophthora thermophila, Pleurotus djamor, Pleurotus ostreatus, Polyporus squamosus, Psathyrella aquatica, Rhizopus microspores, Rhizopus oryzae, Schizophyllum commune, Streptomyces venezuelae, Stropharia rugosoannulata, and Thielavia terrestris.

The mycelium is preferably one or two or more types of mycelia selected from the group including Lentinula edodes, Agaricus arvensis, Armillaria mellea, Flammulina velutipes, Hericium erinaceus, Hypholoma capnoides, Hypholoma sublaterium, Morchella angusticeps, and Polyporus squamosus, is more preferably one or two or more types of mycelia selected from the group including Lentinula edodes, Flammulina velutipes, Hericium erinaceus, Hypholoma capnoides, and Hypholoma sublaterium, and is particularly preferably a mycelium of Lentinula edodes.

Whether the hypha composite material includes the mycelium can be confirmed by detecting α-glucan and β-glucan that are components derived from the mycelium. Syringic acid, vanillic acid, and arabinoxylan, which are the components derived from the mycelium, are components unique to Lentinula edodes, and by detecting these components, it is possible to confirm that the mycelium of Lentinula edodes is included.

### Optional Components

The hypha composite material preferably includes a plasticizer, starch composite particles, and a crosslinking agent as optional components.

### ·Plasticizer

The plasticizer is not particularly limited as long as it is a material capable of imparting flexibility and elasticity to the hypha composite material, and examples thereof include one or more types selected from the group including oil, glycerin, fatliquoring agents, sugar alcohols, diethyloxyester dimethylammonium chloride, non-ionic surfactants (such as Tween 20 and Tween 80), water, glycol, triethyl citrate, acetylated monoglycerides, and epoxidized soybean oil.

The plasticizer used in the embodiment is preferably a sugar alcohol, and the sugar alcohol is preferably one or more types selected from the group including sorbitol, erythritol, and D-mannitol.

### ·Starch Composite Particles

The starch composite particles are a component that functions as a binder that binds the fiber and the mycelium. Since the starch composite particles are biomass-derived raw materials, the starch composite particles can preferably address environmental issues and conserve buried resources.

The starch composite particles are, for example, particles that are obtained by externally plasticizing starch and containing starch and an external plasticizer. The external plasticization of starch can be performed, for example, by mixing a compound having a plurality of functional groups capable of forming a hydrogen bond with a functional group contained in starch in the molecule with starch.

The starch plasticized by the external plasticizer is a high molecular material in which a plurality of α-glucose molecules are polymerized through glycosidic bonds. Examples of starch include amylose and amylopectin.

A weight average molecular weight of starch is not particularly limited, but starch having undergone chemical treatment such as acid treatment and the like and having a polymerization average molecular weight of about 40000 to 600000 may be used.

Examples of the starch can include starch derived from various plants, such as cereals such as corn, wheat, and rice, beans such as broad beans, mung beans, and adzuki beans, tubers such as potato, sweet potato, and tapioca, wild plants such as dogtooth violets, bracken, and kudzu, and palm trees such as sago palm.

As the external plasticizer, for example, a compound having a plurality of functional groups capable of forming a hydrogen bond with a functional group contained in starch in the molecule can be preferably used.

Examples of the functional group capable of forming a hydrogen bond include a hydroxyl group, an amino group, and a carboxyl group.

Examples of the external plasticizer include polyhydric alcohols, such as glycols such as glycerin, ethylene glycol, propylene glycol, diethylene glycol, triethylene glycol, polyethylene glycol, butylene glycol, polyglycerin, and thiodiglycol, sugars such as glucose, fructose, sucrose, galactose, malt sugar, lactose, starch syrup, trehalose, and maltose, sugar alcohols such as sorbitol, maltitol, xylitol, reduced starch syrup, erythritol, mannitol, lactitol, and palatinit, sugar derivatives such as sucralol, hydroxy acids such as tartaric acid, polyvinyl alcohol, trehalose, polyhydroxy(meth)acrylate, and hyaluronic acid, polyvalent amines such as urea and thiourea, polycarboxylic acids such as hyaluronic acid, and polyvinylpyrrolidone. One or two or more types selected from these can be used in combination.

In particular, the external plasticizer preferably contains at least one of polyhydric alcohol, polyvalent amine, and polycarboxylic acids.

A content of the external plasticizer in the starch composite particles is preferably 12% by mass or more and 50% by mass or less, more preferably 15% by mass or more and 40% by mass or less, and still more preferably 20% by mass or more and 30% by mass or less.

The starch composite particle preferably has an average particle diameter of 1 µm or more and 50 µm or less.

### ·Crosslinking Agent

The crosslinking agent is preferably a compound capable of binding fibers via covalent bonds. The term "crosslinking" as used herein includes a bond between fibers and mycelium and a bond between fibers, mycelium, and other components. By adding a crosslinking agent, moisture resistance of the hypha composite material can be improved.

The crosslinking agent is preferably a carboxylic acid, more preferably a dicarboxylic acid or a polycarboxylic acid, and still more preferably a dicarboxylic acid.

The dicarboxylic acid is preferably one or more selected from the group including maleic acid, fumaric acid, oxalic acid, malonic acid, succinic acid, adipic acid, and sebacic acid.

The polycarboxylic acid is preferably one or more selected from the group including citric acid, butanetetracarboxylic acid, and iminodisuccinic acid.

A total content ratio of the plasticizer, the starch composite particles, and the crosslinking agent with respect to the total amount of the hypha composite material is preferably 2.5% by mass or more and 80% by mass or less, and more preferably 3% by mass or more and 70% by mass or less.

When the total content ratio of the plasticizer, the starch composite particles, and the crosslinking agent falls within the above ranges, a hypha composite material having improved flexibility and moisture resistance as well as texture and strength is obtained.

When the hypha composite material includes the plasticizer and the starch composite particles, a content ratio of the plasticizer with respect to a total content (100% by mass) of the plasticizer and the starch composite particles is preferably 10% by mass or more and 80% by mass or less, and more preferably 20% by mass or more and 60% by mass or less.

When the content ratio of the plasticizers falls within the above ranges, a hypha composite material having improved moisture resistance is obtained.

When the hypha composite material includes the starch composite particles and the crosslinking agent, a content ratio of the crosslinking agent with respect to a total content (100% by mass) of the starch composite particles and the crosslinking agent is preferably 1% by mass or more and 50% by mass or less, and more preferably 2% by mass or more and 40% by mass or less.

When the starch composite particles and the crosslinking agent fall within the above ranges, a hypha composite material having improved strength and moisture resistance is obtained.

### Production Method of Hypha Composite Material

A production method of a hypha composite material will be described below in two parts including a production method 1 that is a method for producing a single-layer hypha composite sheet and a production method 2 that is a method for producing a hypha composite material which is a laminate.

### Production Method 1

The production method 1 includes a step of obtaining a fiber base material in which fibers are oriented in a first direction and a step of culturing and growing hyphae using the obtained fiber base material as a medium.

### Step of Obtaining Fiber Base Material

According to the present step, a fiber base material in which the fibers are oriented in the first direction and fiber density is further adjusted is obtained.

Examples of a method for orienting the fibers in the first direction include a method of using an orientation device, a method of pressing the fibers with a roller to be oriented in the first direction, and a method of embedding the fibers with comb teeth to be oriented in the first direction.

The orientation device used in the embodiment will be described with reference to FIG. 2. An orientation device 2 illustrated in FIG. 2 includes a dry defibration unit 21 and a container 23. The dry defibration unit 21 and the container 23 are coupled by a supply tube 22, and the container 23 includes an exhaust tube 24. The supply tube 22 and the exhaust tube 24 are provided on facing walls forming the container 23. With the arrangement of the supply tube 22 and the exhaust tube 24, a gas flow supplied to the container 23 is exhausted in a linear direction indicated by a reference numeral 25.

A wall 23a of the container 23 to which the supply tube 22 is connected is formed of a coarse mesh member having an opening of 1.0 µm or more and 100.0 µm or less. A wall 23b that is a wall facing the wall 23a is formed of a fine mesh member having an opening of 0.9 µm or more and 99 µm or less. The wall 23b includes the exhaust tube 24. The opening of the wall 23a is larger than the opening of the wall 23b.

The dry defibration unit 21 is a dry defibration device provided with a wind generation mechanism. Fiber raw material pellets 20 introduced into the dry defibration unit 21 are defibrated by the dry defibration unit 21 and become defibrated fibers. The defibrated fibers are supplied to the container 23 through the supply tube 22 by a gas flow generated by the wind generation mechanism included in the dry defibration unit 21.

When the defibrated fibers pass through the wall 23a formed of a coarse mesh member, the fibers are aligned in the longitudinal direction by meshes, and only the gas flow passes through the wall 23b formed of the fine mesh members, and is exhausted. Accordingly, fibers 27 aligned in a longitudinal direction 26 are accumulated in the container 23.

In the step of obtaining the fiber base material, the fiber density can be appropriately adjusted according to a desired texture and strength, and is preferably 0.001 g/cm³ or more and 2 g/cm³ or less, more preferably 0.01 g/cm³ or more and 1 g/cm³ or less, and still more preferably 0.1 g/cm³ or more and 0.5 g/cm³ or less.

When a fiber base material having the fiber density in the above ranges is used, hyphae are likely to grow uniformly, and a hypha composite sheet having a uniform texture and strength is easily obtained.

### Step of Culturing and Growing Hyphae Using Fiber Base Material as Medium

The step of culturing and growing hyphae may be performed by using the fiber base material obtained above (the step of obtaining a fiber base material) as a scaffold, adding a medium component, and main-culturing the hyphae on the fiber base material or pre-culturing the hyphae before main culture and then performing main culture.

The pre-culture is an operation in which the desired hyphae are cultured in a pure form in advance to obtain a propagation body before the main culture of the mycelium on the fiber base material.

When the hyphae are pre-cultured, hyphae derived from a selected fungus are inoculated into a known liquid medium or a known solid medium and are cultured. The liquid medium is, for example, an aqueous solution containing medium components such as meat extract and sugar, and the solid medium is an agar medium in which the liquid medium is solidified with agar.

In the case of pre-culture, after the hyphae are inoculated into a liquid medium or a solid medium, the hyphae are cultured in a culture chamber. After confirming elongation of the hyphae, the culture is continued until a desired amount of seed fungus is obtained.

The present culture may be started by inoculating the hyphae in a fiber base material medium in which the medium component is added to the fiber base material, may be started by inoculating the hyphae pre-cultured in the solid medium into the fiber base material medium, or may be started by pouring the hyphae pre-cultured in the liquid medium into the fiber base material together with the liquid medium.

As the medium component, known components can be used, and examples thereof include lignocellulose, simple sugars (such as dextrose and glucose), complex sugars, agar, malt extract, nitrogen sources (such as ammonium nitrate, ammonium chloride, and amino acids), and other minerals (such as magnesium sulfate and phosphate).

Examples of standard agar medium usually used for culturing the mycelium include reinforcement malt extract agar (MEA), potato dextrose agar (PDA), oatmeal agar (OMA), and dog food agar (DFA), and are not limited thereto.

A culture temperature of the main culture may be appropriately adjusted from about 20°C to 40°C in the presence of oxygen depending on the type of fungus.

The main culture is performed under conditions that prevent the formation of fruiting bodies by adjusting light exposure conditions, temperature, carbon dioxide concentration, and the like.

In the main culture step, hyphae are grown with the fibers of the fiber base material as the scaffold, and a hypha composite material in which the fiber and the mycelium are integrated is obtained.

The main culture step is ended when no change in appearance is observed in a top surface direction and a change in thickness in a cross-sectional direction stops (when the thickness becomes saturated).

### Optional Step

The plasticizer, the starch composite particles, and the crosslinking agent may be added to the obtained hypha composite material according to desired strength and texture. Production Method 2

The production method 2 is a method for producing a laminate by laminating the single-layer hypha composite sheet obtained in the above production method 1. The single-layer hypha composite sheets are alternately laminated in a mode in which the orientation directions of the fibers of the single-layer hypha composite sheets intersect. The layers may be integrated by heating and pressurization, or may be integrated by attaching the layers using an adhesive. In addition, the hyphae may be inoculated between the hypha composite sheets and the hyphae may be grown to straddle the hypha composite sheets such that the hypha composite sheets are integrated.

### Examples

According to the present disclosure, the hyphae are grown along a regularly oriented base material.

The hypha composite sheet according to the present disclosure has an appearance with a sense of fiber direction and a fabric-like design, which are created by the orientation of the base material. The hypha composite sheet according to the present disclosure has a smooth touch and strength in the orientation direction.

In contrast, mushroom leather in the related art is obtained by randomly kneading the hypha and the base material.

The mushroom leather in the related art is simply a mixture of materials, resulting in a featureless, solid-colored design. In addition, the mushroom leather in the related art has a rubber-like feel and has an even strength that makes it easy to unravel.

## Claims

1. A hypha composite material comprising:
a fiber oriented in a first direction; and
a mycelium, wherein
the first direction is a longitudinal direction of the fiber, and
the fiber and the mycelium are integrated with cultured and grown hyphae.

2. The hypha composite material according to claim 1, wherein
the hypha composite material is a laminate in which hypha composite sheets are laminated,
at least a first hypha composite sheet and a second hypha composite sheet are laminated, and
an orientation direction of a fiber of the first hypha composite sheet intersects an orientation direction of a fiber of the second hypha composite sheet.

3. The hypha composite material according to claim 1, wherein
the fiber has a number average fiber length of 0.001 mm or more and 5.0 mm or less and a number average fiber diameter of 1.0 µm or more and 100.0 µm or less during defibration.

4. The hypha composite material according to claim 1, wherein
a ratio (A/B) of A to B is 10/90 to 90/10, where A is a content ratio of the fiber and B is a content ratio of the mycelium.

5. The hypha composite material according to claim 1, wherein
the hyphae have a number average fiber diameter of 1 µm or more and 10 µm or less, and the fiber has a number average fiber diameter of 11 µm or more and 30 µm or less.

6. The hypha composite material according to claim 1, wherein
the mycelium is a mycelium of Lentinula edodes.

7. The hypha composite material according to claim 1, further comprising:
a plasticizer.

8. The hypha composite material according to claim 1, further comprising:
a starch composite particle.

9. The hypha composite material according to claim 1, further comprising:
a crosslinking agent.

10. The hypha composite material according to claim 1, wherein
a total content ratio of a plasticizer, a starch composite particle, and a crosslinking agent with respect to a total amount of the hypha composite material is 2.5% by mass or more and 80% by mass or less.

11. The hypha composite material according to claim 1, further comprising:
a sugar alcohol as a plasticizer.

12. The hypha composite material according to claim 1, further comprising:
a sugar alcohol as a plasticizer, wherein
the sugar alcohol is one or more selected from the group including sorbitol, erythritol, and D-mannitol.

13. The hypha composite material according to claim 1, further comprising:
a plasticizer; and
a starch composite particle, wherein
a content ratio of the plasticizer is 10% by mass or more and 80% by mass or less with respect to a total content (100% by mass) of the plasticizer and the starch composite particle.

14. The hypha composite material according to claim 1, further comprising:
a dicarboxylic acid as a crosslinking agent.

15. The hypha composite material according to claim 1, further comprising:
a dicarboxylic acid as a crosslinking agent, wherein
the dicarboxylic acid is one or more selected from the group including succinic acid, adipic acid, and sebacic acid.

16. The hypha composite material according to claim 1, further comprising:
a starch composite particle; and
a crosslinking agent, wherein
a content ratio of the crosslinking agent is 1% by mass or more and 50% by mass or less with respect to a total content (100% by mass) of the starch composite particle and the crosslinking agent.

17. The hypha composite material according to claim 1, further comprising:
a starch composite particle having an average particle diameter of 1 µm or more and 50 µm or less.

18. The hypha composite material according to claim 1, wherein
the fiber contains cellulose.
